# EUROPEAN PATENT APPLICATION

(11) **EP 4 801 238 A1**
(43) Date of publication of application: **02.09.2026**
(21) Application number: 24882509.3
(22) Date of filing: 25.10.2024
(51) Int. Cl.: H10K 30/50, C07D 487/22, H10K 30/40, H10K 30/81, H10K 30/86, H10K 50/14, H10K 85/10, H10K 85/30, H10K 85/50

(54) **PHOTOELECTRIC CONVERSION ELEMENT AND PHOTOELECTRIC CONVERSION DEVICE**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 28.05.2024 JP 2024085999; 23.10.2024 JP 2024186505
(71) Applicant: Canon Kabushiki Kaisha, Tokyo 146-8501 (JP)
(72) Inventor: OHSAWA Tatsuya, Tokyo 146-8501 (JP); NISHIDA Tsutomu, Tokyo 146-8501 (JP); WATARIGUCHI Kaname, Tokyo 146-8501 (JP); KITAHARA Michitaka, Tokyo 146-8501 (JP); MURANAKA Norifumi, Tokyo 146-8501 (JP)
(74) Representative: TBK
(86) International application number: PCT/JP2024/038150
(87) International publication number: WO 2025/089401

(57) **Abstract**

To provide a photoelectric conversion element having improved durability, the present invention is directed to a photoelectric conversion element including: a first electrode; a second electrode; and a photoelectric conversion layer arranged between the first electrode and the second electrode, the photoelectric conversion layer containing a crystal having a perovskite structure, wherein the photoelectric conversion element includes a charge-transporting layer between the photoelectric conversion layer and the first electrode, wherein the charge-transporting layer contains a charge-transporting substance and an insulating resin that are phase-separated from each other, wherein a ratio of a volume of a region of the charge-transporting substance to a volume of a region of the insulating resin is 5 to 30, and wherein, in a binarized image of a two-dimensional spatial distribution of a region A and a region B obtained by measuring the charge-transporting layer with an optical microscope, when a value obtained by Fourier analysis of the binarized image, the value being determined by the following method of calculating D, is represented by D [µm], the D [µm] satisfies 4≤D≤12.

## Description

### [Technical Field]

The present invention relates to a photoelectric conversion element and a photoelectric conversion apparatus.

### [Background Art]

In order to solve a problem of the depletion of fossil energy and a global environmental problem caused by the use of the fossil energy, investigations on a renewable and clean alternative energy source, such as solar energy, wind power, or water power, have been actively performed. In particular, an interest in a solar cell that directly changes sunlight into electrical energy has been increasing. The term "solar cell" as used herein means a battery that generates a current-voltage through utilization of a photovoltaic effect in which light energy is absorbed from sunlight to generate an electron and a hole.

Recently, an n-p diode-type silicon (Si) single crystal-based solar cell having a light energy conversion efficiency of more than 20% has been widely known, and has been actually used in solar power generation. However, the solar cell requires a high temperature treatment step and the price of a material itself is high, and hence there is a problem in that the cost per unit electric power is high. In addition, there is a problem with its supply property in terms of a silicon resource.

Meanwhile, a solar cell using an organic material (hereinafter also referred to as "organic solar cell") does not require the high temperature treatment step, and can be produced in a so-called roll-to-roll system using a sheet-shaped substrate. Accordingly, cost reduction is expected. However, further improvements in power generation efficiency and durability have been desired for practical use of the organic solar cell. In particular, the development of a perovskite solar cell including a crystal having a perovskite structure as a photoelectric conversion layer toward its practical use has been advanced because the cell is excellent in photoelectric conversion characteristic. For example, in Patent Literature 1, there is a description of a technology including incorporating an organic semiconductor and a polymer compound having a glass transition temperature of 100°C or more into a hole-transporting layer to improve its peeling from an electrode. In Non Patent Literature 1, there is a description of a technology of a charge-transporting layer for an inverted layer that improves photoelectric conversion efficiency by doping PEDOT:PSS with nickel phthalocyanine having a substituent.

### [Citation List]

### [Patent Literature]

PTL 1: Japanese Patent Laid-Open No. 2018-170382

### [Non Patent Literature]

NPL 1: Xian-Fu Zhang, et al, J. Mater. Chem. A, 2018, 6, 12515-12522

### [Summary of Invention]

### [Technical Problem]

According to investigations made by the inventors of the present invention, it has been found that there is room for improvement in durability in each of the photoelectric conversion elements described in Patent Literature 1 and Non Patent Literature 1.

Accordingly, the present invention is directed to providing a photoelectric conversion element having improved durability. The present invention is also directed to providing a photoelectric conversion apparatus.

### [Solution to Problem]

The above-mentioned provision is achieved by the present invention described below. That is, the present invention is directed to
a photoelectric conversion element including: a first electrode; a second electrode; and a photoelectric conversion layer arranged between the first electrode and the second electrode, the photoelectric conversion layer containing a crystal having a perovskite structure,
wherein the photoelectric conversion element includes a charge-transporting layer between the photoelectric conversion layer and the first electrode,
wherein the charge-transporting layer contains a charge-transporting substance and an insulating resin that are phase-separated from each other,
wherein a ratio of a volume of a region of the charge-transporting substance to a volume of a region of the insulating resin is 5 to 30, and
wherein, in a binarized image of a two-dimensional spatial distribution of a region A and a region B obtained by measuring the charge-transporting layer with an optical microscope, when a value obtained by Fourier analysis of the binarized image, the value being determined by the following method of calculating D, is represented by D [µm], the D [µm] satisfies the following formula (E1). $4 \leq D \leq 12$

### <Method of calculating D>

With regard to the region A and the region B, an image of a two-dimensional spatial distribution having a size of L [µm]×L [µm], where L [µm] is 100 µm or more, is binarized by Otsu's binarization method as shown in the following formula (E3):
[Math. 1] $c \left[m, n\right] = \left\{\begin{matrix}1 & for region A \\ 0 & for region B\end{matrix}\right.$ where, for discrete data of N×N pixels obtained by dividing L into N with an even number N of 1,024 or more, "m" and "n" each represent an integer of -N/2 to N/2-1, and each represent a coordinate in the two-dimensional spatial distribution.

Next, c[m, n] obtained by the formula (E3) is subjected to discrete Fourier transform in accordance with the following formula (E4), where "k" and "l" each represent an integer of -N/2 to N/2.
[Math. 2] $C \left[k, l\right] = {\sum }_{m = - N / 2}^{N / 2 - 1} {\sum }_{n = - N / 2}^{N / 2 - 1} c \left[m, n\right] {e}^{- i \frac{2 π}{N} \left(km+ln\right)}$

Then, a domain size D [µm] is calculated in accordance with the following formula (E5) using C[k, l] obtained by the formula (E4).
[Math. 3] $D = \frac{L \left[{\sum }_{\begin{matrix}k , l = - \frac{N}{2} \\ \left(k, l\right) \neq \left(0, 0\right)\end{matrix}}^{\frac{N}{2} - 1} \frac{C \left[k, l\right] C \left[-k,-l\right]}{\sqrt{{k}^{2} + {l}^{2}}}\right]}{\left[{\sum }_{\begin{matrix}k , l = - \frac{N}{2} \\ \left(k, l\right) \neq \left(0, 0\right)\end{matrix}}^{\frac{N}{2} - 1} \left(C\left[k, l\right]C\left[-k,-l\right]\right)\right]}$

### [Advantageous Effects of Invention]

According to the present invention, the photoelectric conversion element having improved durability can be provided.

### [Brief Description of Drawings]

[Fig. 1]

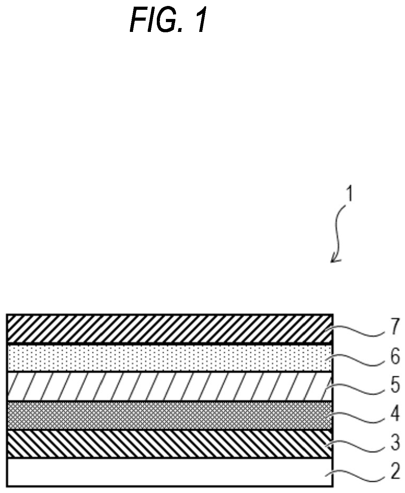

   Fig. 1 is a schematic sectional view in a thickness direction of a photoelectric conversion element of the present invention.
[Fig. 2]

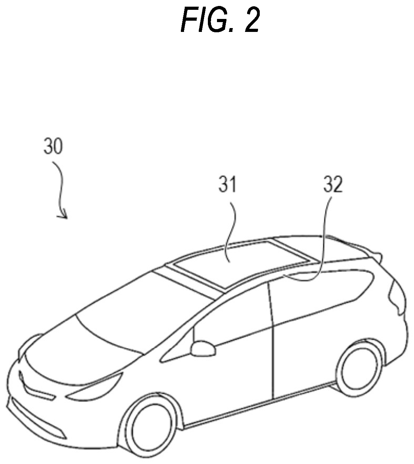

   Fig. 2 is a perspective view for schematically illustrating an example of a moving body including the photoelectric conversion element of the present invention.
[Fig. 3]

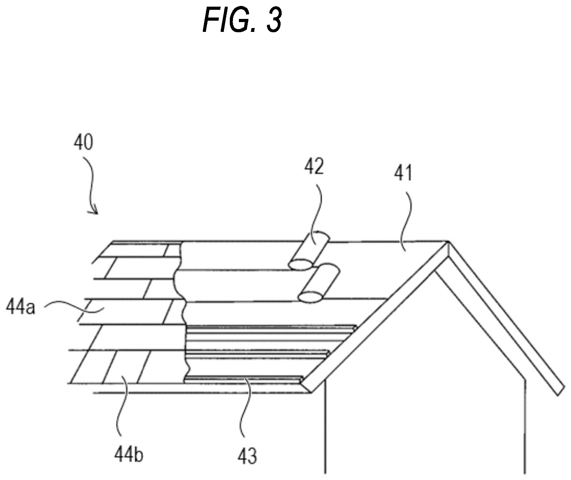

   Fig. 3 is a perspective view for schematically illustrating an example of a building material including the photoelectric conversion element of the present invention.
[Fig. 4]

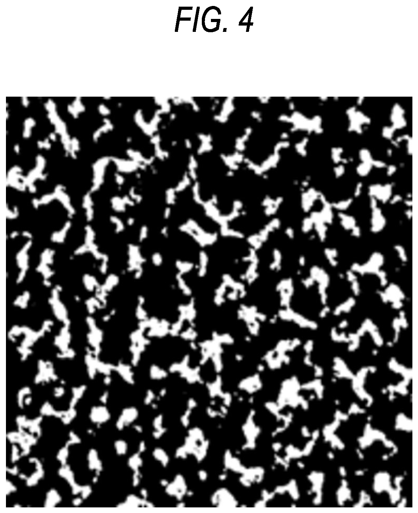

   Fig. 4 is an example of a binarized image obtained in Example 1 of the present invention.

### [Description of Embodiments]

### <One Embodiment>

One embodiment is directed to a photoelectric conversion element.

A photoelectric conversion element of the present invention is
a photoelectric conversion element including: a first electrode; a second electrode; and a photoelectric conversion layer arranged between the first electrode and the second electrode, the photoelectric conversion layer containing a crystal having a perovskite structure,
wherein the photoelectric conversion element includes a charge-transporting layer between the photoelectric conversion layer and the first electrode,
wherein the charge-transporting layer contains a charge-transporting substance and an insulating resin that are phase-separated from each other,
wherein a ratio of a volume of a region of the charge-transporting substance to a volume of a region of the insulating resin is 5 to 30, and
wherein, in a binarized image of a two-dimensional spatial distribution of a region A and a region B obtained by measuring the charge-transporting layer with an optical microscope, when a value obtained by Fourier analysis of the binarized image, the value being determined by the following method of calculating D, is represented by D [µm], the D [µm] satisfies the following formula (E1). $4 \leq D \leq 12$

### <Method of calculating D>

With regard to the region A and the region B, an image of a two-dimensional spatial distribution having a size of L [µm]×L [µm], where L [µm] is 100 µm or more, is binarized by Otsu's binarization method as shown in the following formula (E3):
[Math. 4] $c \left[m, n\right] = \left\{\begin{matrix}1 & for region A \\ 0 & for region B\end{matrix}\right.$ where, for discrete data of N×N pixels obtained by dividing L into N with an even number N of 1,024 or more, "m" and "n" each represent an integer of -N/2 to N/2-1, and each represent a coordinate in the two-dimensional spatial distribution.

Next, c[m, n] obtained by the formula (E3) is subjected to discrete Fourier transform in accordance with the following formula (E4), where "k" and "l" each represent an integer of -N/2 to N/2.
[Math. 5] $C \left[k, l\right] = {\sum }_{m = - N / 2}^{N / 2 - 1} {\sum }_{n = - N / 2}^{N / 2 - 1} c \left[m, n\right] {e}^{- i \frac{2 π}{N} \left(km+ln\right)}$

Then, a domain size D [µm] is calculated in accordance with the following formula (E5) by using C[k, l] obtained by the formula (E4).
[Math. 6] $D = \frac{L \left[{\sum }_{\begin{matrix}k , l = - \frac{N}{2} \\ \left(k, l\right) \neq \left(0, 0\right)\end{matrix}}^{\frac{N}{2} - 1} \frac{C \left[k, l\right] C \left[-k,-l\right]}{\sqrt{{k}^{2} + {l}^{2}}}\right]}{\left[{\sum }_{\begin{matrix}k , l = - \frac{N}{2} \\ \left(k, l\right) \neq \left(0, 0\right)\end{matrix}}^{\frac{N}{2} - 1} \left(C\left[k, l\right]C\left[-k,-l\right]\right)\right]}$

Measurement conditions for the optical microscope are shown below.

### <Measurement Conditions for Optical Microscope>

Apparatus name: OPTELICS
HYBRID
L3 (manufactured by Lasertec Corporation)
Software: LMeye7
Lens: 100X/0.95
OFN25
WD0.32 (Nicon Corporation)
Light source: white light
CCD mode: selected in accordance with a measurement target
Scan rate: standard
Image size: 1,024×1,024
Operation: normal
Exposure time: standard
Confocal: ON

### <Meaning of D>

In the description of the meaning of the D, the continuous variable representation of the formula (E4) is shown in the following formula (E7).
[Math. 7] $C \left(\vec{q}\right) = \int {\int }_{- ∞}^{∞} c \left(\vec{r}\right) {e}^{- i 2 π \vec{q} ⋅ \vec{r}} d \vec{r}$

In the formula, $c \left(\vec{r}\right)$ corresponds to the continuous variable representation of the formula (E3), and the position vector: $\vec{r}$ may be written as $\vec{r} = \left(x, y\right)$ . The (x, y) is the continuous variable representation (continuous coordinates) of the discrete coordinates [m, n]. In addition, the wavenumber vector: $\vec{q}$ may be written as $\vec{q} = \left({q}_{x}, {q}_{y}\right)$ . The (qₓ, q_{y}) corresponds to [k, l] in the discrete representation.

The $C \left(\vec{q}\right)$ defined by the formula (E7) is the continuous Fourier transform of $c \left(\vec{r}\right)$ , which is the continuous variable representation of the original binarized data. Accordingly, the square of the absolute value thereof: ${\left‖C\left(\vec{q}\right)\right‖}^{2}$ is called a so-called power spectrum and is calculated by $C \left[k, l\right] C \left[-k,-l\right]$ in the discrete variable representation. In addition, the reciprocal of the absolute value of the wavenumber vector: $1 / \left‖\vec{q}\right‖$ has a distance dimension and is calculated by $\frac{1}{\sqrt{{k}^{2} + {l}^{2}}}$ in the discrete variable representation. In view of the above-mentioned correspondence, the continuous variable representation corresponding to the formula (E5) is the following formula (E8).
[Math. 19] $D = \frac{{\int \int }_{- ∞}^{∞} {\left‖C\left(\vec{q}\right)\right‖}^{2} / \left‖\vec{q}\right‖ d \vec{q}}{{\int \int }_{- ∞}^{∞} {\left‖C\left(\vec{q}\right)\right‖}^{2} d \vec{q}}$

As is clear from the formula (E8), the D in the continuous variable representation is a value obtained by averaging the value having a distance dimension: $1 / \left‖\vec{q}\right‖$ , with respect to the variable: $\vec{q}$ , with the weighting of the power spectrum: ${\left‖C\left(\vec{q}\right)\right‖}^{2}$ . The power spectrum: ${\left‖C\left(\vec{q}\right)\right‖}^{2}$ obtained by the Fourier transform has a large value at a wavenumber "q" corresponding to a spatial period characteristic of a system of interest, and hence the weighted average value D obtained by the formula (E8) means a representative value of the length of the spatial period characteristic of the system.

The fact that the D has the above-mentioned meaning is the same in the discrete variable representation. In this case, the above-mentioned system of interest is a binarized image, and hence the D calculated by the formula (E5) means a representative value of the length of the spatial period characteristic of the binarized image. Accordingly, on one hand, when the D is small, the mesh of the two-dimensional spatial distribution formed by the region A and the region B of the binarized image is fine, and on the other hand, when the D is large, the mesh of the two-dimensional spatial distribution is coarse.

In the sum in the wavenumber space [k, l] of the formula (E5), the origin [k, l]=[0, 0] is excluded. This is because the value at the origin of the discrete Fourier transform C[k, l] has information on an area ratio between the region A and the region B in the original binarized image, and only the spatial period of the two-dimensional spatial distribution formed by the region A and the region B is required as information for the D in the present invention.

As a result of investigations, the inventors of the present invention have found that, when the above-mentioned configuration is satisfied, the interlayer migration of an organic anion in the perovskite crystal of the photoelectric conversion layer can be suppressed, and hence the durability of the photoelectric conversion element can be improved. It has hitherto been known that an organic anion in a photoelectric conversion layer migrates toward an anode electrode side to break a photoelectric conversion element. Accordingly, the inventors have arranged a charge-transporting layer between the photoelectric conversion layer and the anode electrode. The charge-transporting layer contains a charge-transporting substance and an insulating resin, and phase separation was observed by observation with an optical microscope. In order to evaluate the size of the phase separation, the value D determined by the formula was used. As a result, when the ratio of the volume of the region of the charge-transporting substance to the volume of the region of the insulating resin is 5 to 30 and the D [µm] satisfies the formula (E1), the breakage of the photoelectric conversion element was able to be suppressed. The inventors have presumed the reason for this to be described below.

When appropriate amounts of the insulating resin and the charge-transporting substance are phase-separated, a strong electrical interaction acts between the insulating resin, through which a charge carrier is difficult to flow when the carrier is generated, and the charge-transporting substance, through which the carrier is easy to flow, resulting in an imbalance in charge distribution. It is conceived that the imbalance in charge suppresses the migration of the organic anion in the photoelectric conversion layer, and as a result, the durability of the photoelectric conversion element is improved in the above-mentioned configuration. In this mechanism, the distribution of the charge imbalance needs to be sufficiently fine so that the organic anion is sufficiently captured by the charge imbalance and durability degradation due to migration is suppressed. As a result of investigations made by the inventors of the present invention from this viewpoint, it has been required to satisfy the condition represented by the formula (E1). $4 \leq D \leq 12$ As described above in the section <Meaning of D>, when the D is larger than 12, the mesh of the two-dimensional spatial distribution becomes coarse, and hence the distribution of the charge imbalance generated by the electrical interaction between the region A and the region B in the two-dimensional spatial distribution also becomes coarse, and the organic anion passes through a part where no charge imbalance is present to cause migration, resulting in the progress of durability degradation of the photoelectric conversion element. In contrast, when the D is smaller than 4, the region A and the region B are in a well-mixed state, and hence the distinction between the insulating resin region and the charge-transporting substance region becomes ambiguous. Accordingly, the electrical interaction acting between both the regions becomes weak, and hence the organic anion cannot be sufficiently captured.

In the binarized image of the two-dimensional spatial distribution of the region A and the region B obtained by measuring the charge-transporting layer with the optical microscope, when a value obtained by Fourier analysis of the binarized image, the value being determined by the following method of calculating P, is represented by P, the P preferably satisfies the following formula (E2). $8.0 \leq P \leq 9.7$

### <Method of calculating P>

An order parameter P is calculated in accordance with the following formula (E6) by using C[k, l] obtained by the formula (E4).
[Math. 24] $P = - {\sum }_{\begin{matrix}k , l = - N / 2 \\ \left(k, l\right) \neq \left(0, 0\right)\end{matrix}}^{N / 2 - 1} \left\{\left[\begin{matrix}C \left[k, l\right] C \left[-k,-l\right] \\ / \left({\sum }_{\begin{matrix}k , l = - N / 2 \\ \left(k, l\right) \neq \left(0, 0\right)\end{matrix}}^{N / 2 - 1} \left(C\left[k, l\right]C\left[-k,-l\right]\right)\right)\end{matrix}\right]ln\left[\begin{matrix}C \left[k, l\right] C \left[-k,-l\right] \\ / \left({\sum }_{\begin{matrix}k , l = - N / 2 \\ \left(k, l\right) \neq \left(0, 0\right)\end{matrix}}^{N / 2 - 1} \left(C\left[k, l\right]C\left[-k,-l\right]\right)\right)\end{matrix}\right]\right\}$

### <Meaning of P>

In the description of the meaning of the P, the continuous variable representation of the formula (E6) is shown in the following formula (E9).
[Math. 25] $P = - {\int \int }_{- ∞}^{∞} \left[\frac{{\left‖C\left(\vec{q}\right)\right‖}^{2}}{{\int \int }_{- ∞}^{∞} {\left‖C\left(\vec{q}\right)\right‖}^{2} d \vec{q}}ln\frac{{\left‖C\left(\vec{q}\right)\right‖}^{2}}{{\int \int }_{- ∞}^{∞} {\left‖C\left(\vec{q}\right)\right‖}^{2} d \vec{q}}\right] d \vec{q}$

In the formula (E9), ${\left‖C\left(\vec{q}\right)\right‖}^{2}$ is a power spectrum as described above, and has a large value at a wavenumber "q" corresponding to a spatial period characteristic of a system of interest. Accordingly, a value: ${\left‖C\left(\vec{q}\right)\right‖}^{2} / \int {\int }_{- ∞}^{∞} {\left‖C\left(\vec{q}\right)\right‖}^{2} d \vec{q}$ , which is obtained by dividing the power spectrum by its integral in the wavenumber space, is a weighting function that adopts a value of 0 to 1. The formula (E9) calculates Shannon entropy by identifying this weighting function with probability, and hence the P calculated by the formula (E9) corresponds to a parameter representing the degree of order of the original binarized image, that is, entropy.

When the P falls within the range that satisfies the formula (E2), the migration of the organic anion can be further suppressed.

With regard to the photoelectric conversion element of the present invention, the charge-transporting substance is preferably a pigment having a particle diameter of 1.0×10¹ to 5.0×10² nm. When the particle diameter falls within the above-mentioned range, a charge distribution that can suppress the migration is easily formed. In addition, specific examples of the pigment include a phthalocyanine pigment, an azo pigment, a lake pigment, a quinacridone pigment, a dioxazine pigment, a perylene pigment, and an isoindolinone pigment. The particle diameter may be selected by, for example, the selection of the kind of the insulating resin, the ratio (volume ratio) of the volume of the charge-transporting substance to the volume of the insulating resin, the combined use of a dispersant or dispersion conditions during dispersion, or the performance of centrifugation.

In the photoelectric conversion element of the present invention, it is more preferred that the charge-transporting substance be a phthalocyanine compound, and it is still more preferred that the phthalocyanine compound have a structure represented by the following formula (Pc-2). The charge-transporting substance can more efficiently form the charge distribution that suppresses the migration. 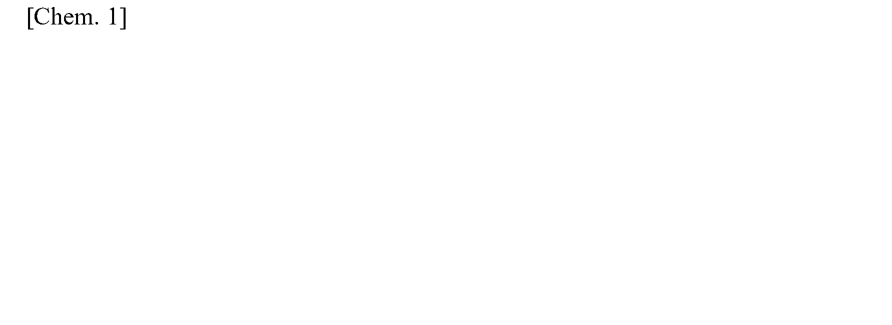 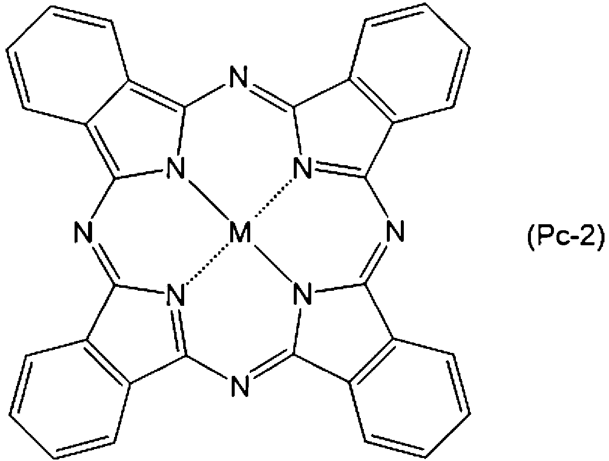 M in the formula (Pc-2) represents H₂, a metal atom having a ligand, or a metal atom free of a ligand. In the present invention, the structure of a chemical substance may be determined by nuclear magnetic resonance (NMR).

In particular, when M in the formula (Pc-2) represents H₂, the formula (Pc-2) is represented by the following formula (Pc-1).

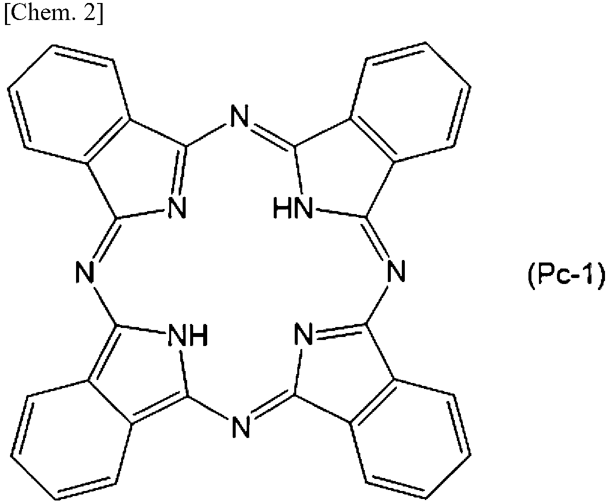

Specific examples of the insulating resin include a polyacetal resin, an acrylic resin, a polyarylate resin, a polycarbonate resin, a polyvinyl acetate resin, a polyester resin, a polyamide resin, a polyurethane resin, and a polystyrene resin. The molecular weight of the insulating resin preferably falls within the range of 1,000 to 1,000,000 in terms of weight-average molecular weight.

In the photoelectric conversion element of the present invention, the glass transition temperature of the insulating resin is preferably 95°C or less. When the glass transition temperature falls within this range, the insulating resin is easily brought into close contact with the charge-transporting substance, and hence a more effective charge distribution can be formed. The glass transition temperature may be determined with a differential scanning calorimeter (DSC).

In the photoelectric conversion element of the present invention, the insulating resin is preferably a polyvinyl acetal resin or a polyvinyl butyral resin. The above-mentioned insulating resin is easily brought into close contact with the charge-transporting substance, and hence a more effective charge distribution can be formed.

In the photoelectric conversion element of the present invention, it is preferred that the charge-transporting layer contain an aromatic ring compound having a hydroxy group, the aromatic ring compound being different from the charge-transporting substance (pigment) and the insulating resin. When the charge-transporting layer contains the aromatic ring compound having a hydroxy group, the charge-transporting substance and the insulating resin are easily brought into contact with each other, and hence a more effective charge distribution can be formed.

In order to control the D so as to satisfy the range of the formula (E1): 4≤D≤12 in the present invention, it is required to appropriately select the thickness of the charge-transporting layer, the kind of the charge-transporting substance, the kind of the insulating resin, and the like in addition to the ratio of the volume of the charge-transporting substance to the volume of the insulating resin. In addition, when the charge-transporting substance is a pigment, the setting of the particle diameter thereof to 1.0×10¹ to 5.0×10² nm is one effective means for satisfying the range of the formula (E1).

Further, in order to control the P so as to satisfy the range of the formula (E2): 8.0≤P≤9.7 in the present invention, it is required to appropriately select means for forming the charge-transporting layer. For example, when the charge-transporting layer is formed by spin coating, the selection of a distance between a place where a coating liquid is dropped and a place of the photoelectric conversion element of the present invention is given as one example. As the place of the photoelectric conversion element of the present invention becomes farther from the place where the coating liquid is dropped by the spin coating, the two-dimensional spatial distribution of the region A and the region B tends to have anisotropy by centrifugal force, and hence the order tends to increase and the P tends to reduce.

The photoelectric conversion element of the present invention may include a second charge-transporting layer between the first electrode and the charge-transporting layer. When the photoelectric conversion element includes the second charge-transporting layer, the transfer of a carrier to an electrode may be facilitated.

The effects of the present invention can be achieved when the respective configurations synergistically exhibit effects on each other as in the mechanism as described above.

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of a constituent element gradually changes, or a layer that may form a complicatedly intricate structure together with another layer. In addition, the elemental analysis of the layer may be performed by, for example, performing the TOF-SIMS/FE-TEM/EDS line analysis measurement of a cross section of the photoelectric conversion element and observing the element distribution of a specific element. The analysis of each layer may be performed by peeling and removing a layer from a completed photoelectric conversion element to expose the layer to be analyzed. In the present invention, for the quantification of a volume ratio, the area ratio of an exposed surface or cross section is used as the volume ratio of the layer.

Fig. 1 is a sectional view for schematically illustrating the configuration of the photoelectric conversion element according to one embodiment of the present invention. A photoelectric conversion element 1 includes a substrate 2, and a second electrode 3, an electron-transporting layer 4, a photoelectric conversion layer 5, a charge-transporting layer 6, and a first electrode 7 arranged thereon. One of the first electrode 7 and the second electrode 3 is an anode, and the other is a cathode. A current can be extracted by connecting the first electrode 7 and the second electrode 3 with an external circuit.

The photoelectric conversion layer 5 is excited by light that has entered the layer through the substrate 2, the second electrode 3, and the electron-transporting layer 4, or the first electrode 7 and the charge-transporting layer 6 to generate an electron or a hole. That is, the photoelectric conversion layer 5 generates a current between the first electrode 7 and the second electrode 3. The electron-transporting layer 4 is a layer arranged between the photoelectric conversion layer 5, and the two electrodes (the second electrode 3 and the first electrode 7), and may not be formed in some cases. A form in which the plurality of electron-transporting layers 4 and photoelectric conversion layers 5 are laminated may be adopted. Such form may also be referred to as "tandem structure." The respective members are described below. In addition, the photoelectric conversion element may be produced in the order of the first electrode 7, the charge-transporting layer 6, the photoelectric conversion layer 5, the electron-transporting layer 4, and the second electrode 3 on the substrate 2.

### [Photoelectric Conversion Element]

The photoelectric conversion element of the present invention is characterized by including: the first electrode; the second electrode; the photoelectric conversion layer arranged between the first electrode and the second electrode, the photoelectric conversion layer containing the crystal having a perovskite structure; and the charge-transporting layer between the photoelectric conversion layer and the first electrode. In addition, in order to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a perovskite crystal in its photoelectric conversion layer.

A method of forming each of the layers including the photoelectric conversion layer and charge-transporting layer of the photoelectric conversion element of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with each layer.

The respective layers are described below.

### [Substrate]

The photoelectric conversion element 1 of the present invention may include the substrate 2, and examples thereof include a transparent glass substrate made of soda-lime glass or alkali-free glass, a ceramic substrate, and a transparent plastic substrate. When light is taken in from the first electrode 7 side, an opaque material may be used as the substrate 2, and when light is taken in from the second electrode 3 side, the substrate 2 is formed of a transparent material.

### [Electrode]

A material for the first electrode 7 or the second electrode 3 is not particularly limited, and a material that has hitherto been known may be used. Examples thereof include: metals, such as gold, silver, titanium, and copper; sodium; a sodium-potassium alloy; lithium; magnesium; carbon; aluminum; a magnesium-silver mixture; a magnesium-indium mixture; a carbon nanotube; an aluminum-lithium alloy; an Al/Al₂O₃ mixture; and an Al/LiF mixture. Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers. Those materials may be used alone or in combination thereof. At least one electrode of the first electrode 7 or the second electrode 3 on a light incident side is a transparent electrode, and the other may be a transparent electrode or may also serve as a reflective layer formed of a light reflective material, or may be a transparent electrode including a reflective layer on a side opposite to the light incident side. When the first electrode 7 is on the light incident side, the second electrode 3 and the substrate 2 may be a transparent electrode and a reflective layer, respectively. The electrode may be a patterned electrode.

### [Photoelectric Conversion Layer]

The photoelectric conversion layer 5 contains the crystal having a perovskite structure. The crystal having a perovskite structure to be used in the present invention is preferably represented by the following general formula [1].

AₒBₚX_{q} [1]

In the general formula [1], A represents a cation, B represents a cation, and X represents an anion.

"o", "p", and "q" satisfy 0≤o≤10, 0≤p≤10, and 0≤q≤20, respectively, and A, B, and X may each be formed of a single material, or a combination of two or more kinds of materials. An additive may be added to the extent that the general formula is satisfied. The general formula generally forms a perovskite crystal having a three-dimensional structure, but when the cation A to be formed is large enough to fit within a crystal having a three-dimensional perovskite structure, a crystal having a two-dimensional perovskite structure, a crystal having a 2.5-dimensional perovskite structure having both the properties of two-dimensional and three-dimensional perovskite structures, a two-layer crystal having three-dimensional and two-dimensional perovskite structures, or a crystal having a mixed three-dimensional/two-dimensional perovskite structure is formed, and each of the crystals functions as a photoelectric conversion layer. The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed. The crystal having a two-dimensional perovskite or 2.5-dimensional perovskite structure may form a perovskite structure of a Ruddlesden-Popper (RP) type, a Dion-Jacobson (DJ) type, or an alternating cations in the interlayer (ACI) type.

The cation A of the general formula [1] is not particularly limited. The cation A may or may not have a substituent, and specific examples thereof include the following formulae (C1) to (C67).

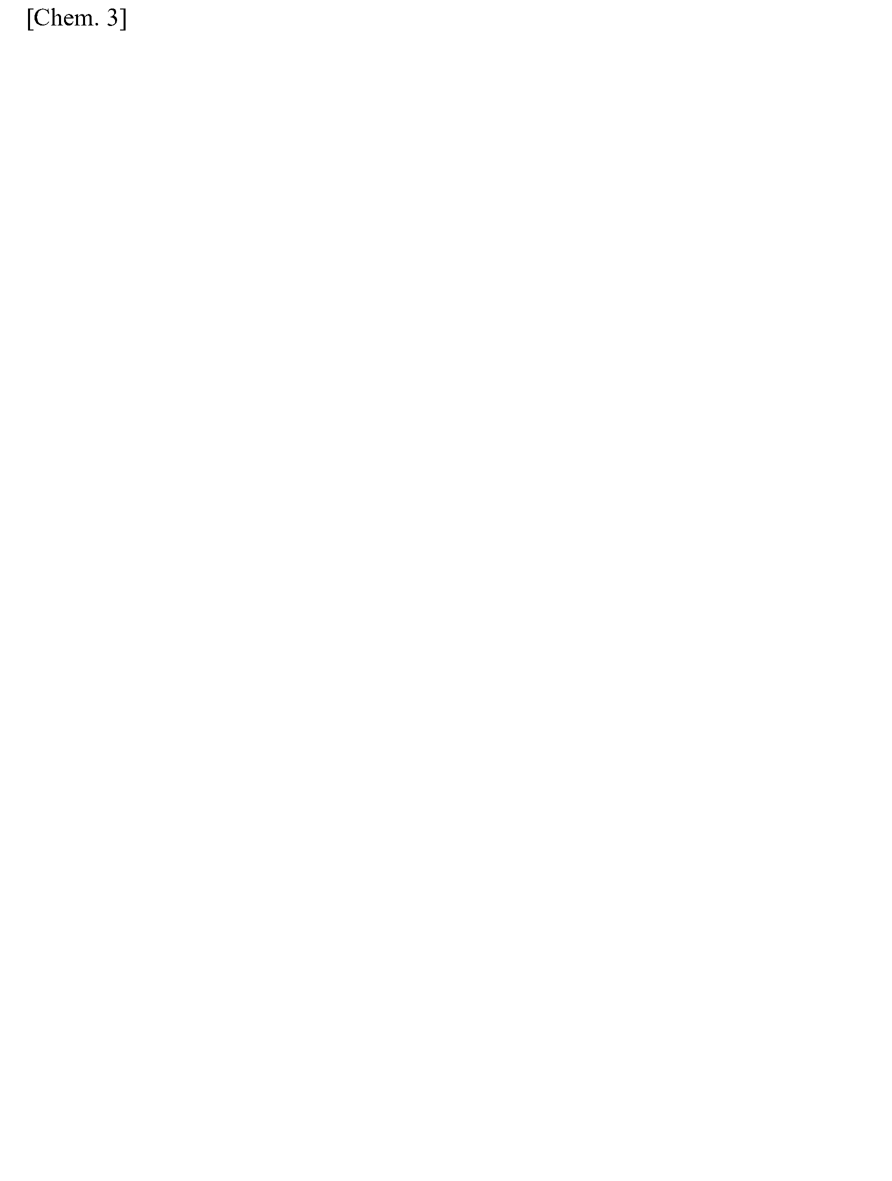

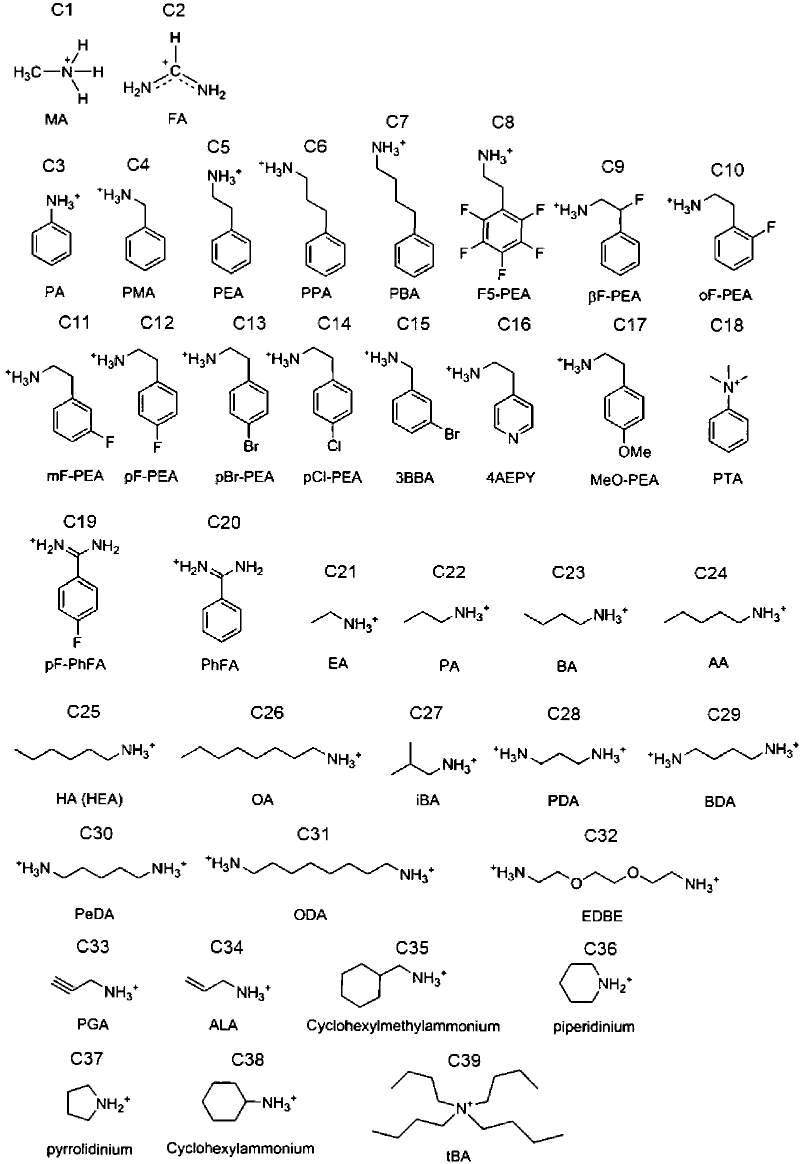

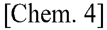

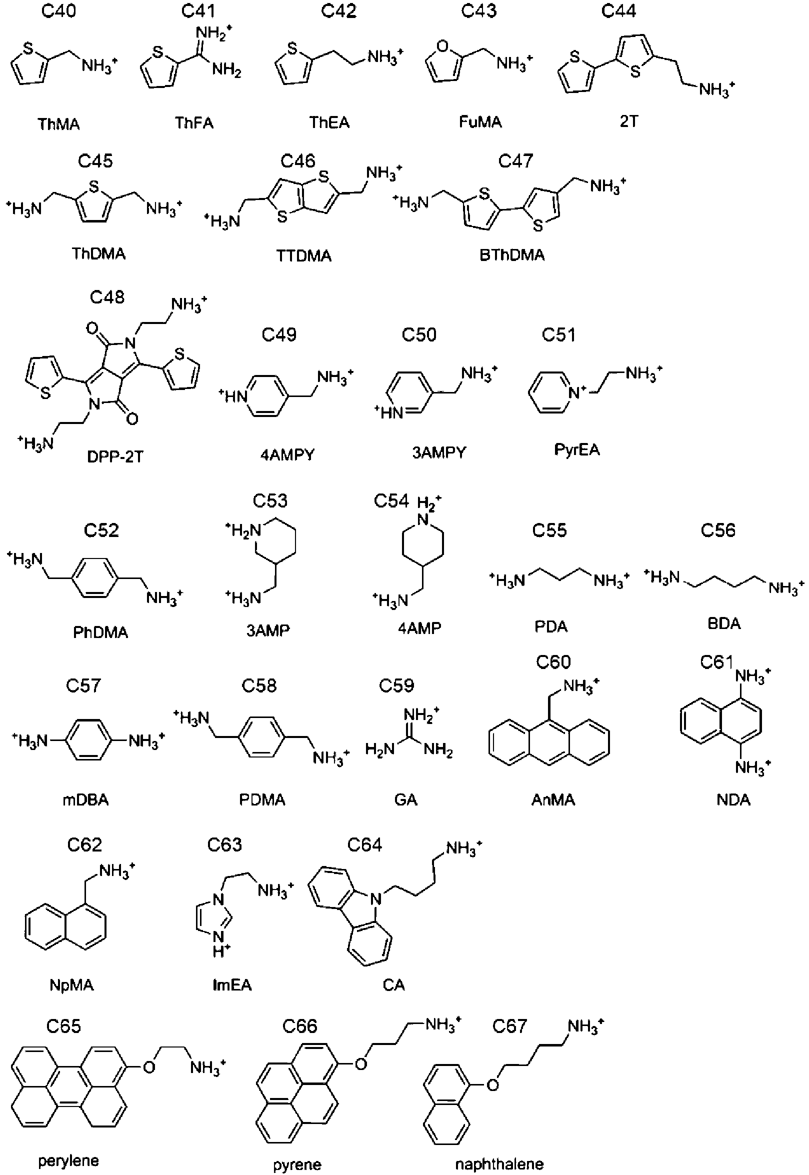

In addition, an inorganic atom is not particularly limited, and lithium, cesium, sodium, potassium, and rubidium are preferred. Those organic molecules or inorganic atoms may be used alone or in combination thereof.

B in the general formula [1] represents a cation atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, bismuth, and silver are preferred from the viewpoint of the stability of a perovskite crystal structure. Those atoms may be used alone or in combination thereof.

X in the general formula [1] represents a halogen or chalcogen atom, and examples thereof include chlorine, bromine, iodine, oxygen, sulfur, selenium, tellurium, and polonium. Those halogen or chalcogen atoms may be used alone or in combination thereof. Of those, a halogen atom is preferred because, when the halogen atom is incorporated into the structure, the above-mentioned crystal having a perovskite structure easily becomes soluble in an organic solvent, and hence its application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Iₓ₄Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (pF-PEA)₂(FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (BA_{0.9}PEA_{0.1})₂MA₄Pb₅I₁₆, (BA_{0.9}PEA_{0.1})₂MA₃Pb₄I₁₃, (BA)₂MA₂Pb₃I₁₀, (BA)₂MA₃Pb₄I₁₃, (BA)₂MA₄Pb₅I₁₆, (BA)₂MA₃Pb₄I₁₃, CsSnBr₃, CsSnI₃, FA_{0.75}MA_{0.25}Sn_{0.95}Ge_{0.05}I₃, FAMASnGeI₃, FASnBr₃, FASnI₃, MA₂Sn₃I₈, MASnBr₃, MASnGeI₃, and MASnI₃ are preferred.

The A site, B site, or X site of each of the general formulae may be adjusted to be deficient or excessive in accordance with purposes, and the combinations of x1 to x5 may be changed in accordance with purposes. Examples of the combinations of x1 to x5 are as shown in Table 1. Particularly preferred ranges are 0.03≤x1≤0.10, 0.80≤x2≤0.96, 0.95≤x3≤1.05, 0.80≤x4≤0.96, and 2.95≤x5≤3.05. MACl may be included as a material for forming a perovskite crystal.

### [Table 1]

**Table 1**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

An organic-inorganic perovskite compound to be used in the present invention is preferably a crystalline semiconductor. The term "crystalline semiconductor" means a semiconductor that enables the measurement of an X-ray scattering intensity distribution to detect a scattering peak. When the organic-inorganic perovskite compound is the crystalline semiconductor, the mobility of the electron in the organic-inorganic perovskite compound increases, and the photoelectric conversion efficiency of the photoelectric conversion element is improved.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm or more, a more preferred upper limit is 1,200 nm, a still more preferred lower limit is 100 nm, and a still more preferred upper limit is 1,000 nm.

### [Charge-transporting Layer]

In the photoelectric conversion element of the present invention, the charge-transporting layer includes a region of a charge-transporting substance and a region of an insulating resin that are phase-separated from each other, and the ratio of the volume of the region of the charge-transporting substance to the volume of the region of the insulating resin is 5 to 30. In the present invention, the charge-transporting layer contains a charge-transporting substance that is a P-type semiconductor and an insulating resin. The insulating resin has a volume resistivity of 10⁸ Ω·cm or more. As a method of measuring a ratio between the volume of the region of the insulating resin and the volume of the region of the charge-transporting substance, determination may be performed from, for example, an area ratio of a cross section determined by FE-TEM/EDS as described above.

The thickness of the charge-transporting layer is preferably 1 to 1,000 nm, more preferably 5 to 500 nm, particularly preferably 10 to 200 nm.

The charge-transporting layer may be formed by preparing a coating liquid for a charge-transporting layer containing the above-mentioned respective materials and a solvent, forming a coating film of the liquid on the photoelectric conversion layer, and drying the coating film. Examples of the solvent to be used for the coating liquid include an alcohol-based solvent, a ketone-based solvent, an ether-based solvent, an ester-based solvent, and an aromatic hydrocarbon-based solvent. Of those solvents, an alcohol-based solvent or an aromatic hydrocarbon-based solvent is preferred.

### [Second Charge-transporting Layer]

In the present invention, the photoelectric conversion element 1 may further include the second charge-transporting layer between the charge-transporting layer 6 and the first electrode 7 from the viewpoint of the compatibility of a film of the charge-transporting layer 6.

A material for the second charge-transporting layer is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, a thiocyanate compound, and a thiophene compound. The compound particularly preferably has an aromatic ring from the viewpoint of the compatibility of a film interface, and Spiro-OMeTAD, PTAA, or a phthalocyanine compound is preferred.

In addition, the second charge-transporting layer may contain a dopant as an additive in order to improve its charge transportation capability. Examples of a substance that may be used as the dopant include lithium compounds such as lithium bis(trifluoromethanesulfonyl)imide, cobalt compounds such as [tris(2-(1H-pyrazol-1-yl)-4-tert-butylpyridine)cobalt(III) tris(bis(trifluoromethylsulfonyl)imide)], boron compounds such as tetrakis(pentafluorophenyl)borate, molybdenum compounds such as tris[1-(methoxycarbonyl)-2-(trifluoromethyl)-ethane-1,2-dithiolene]molybdenum, organic compounds each having a tetracyanoquinodimethane skeleton such as 2,3,4,6-tetrafluoro-7,7,8,8-tetracyanoquinodimethane, and organic compounds each having a pyridine skeleton such as 4-tert-butylpyridine.

### [Electron-transporting Layer]

In the photoelectric conversion element of the present invention, the electron-transporting layer 4 may be arranged between the second electrode 3 and the photoelectric conversion layer 5 as illustrated in Fig. 1.

A material for the electron-transporting layer 4 is not particularly limited, and examples thereof include an N-type conductive polymer, an N-type low-molecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a fullerene compound, a perylene derivative, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide. Specifically, tin oxide may be obtained by causing tin(II) chloride, tin(IV) chloride, tin(II) chloride dihydrate, or tin(IV) chloride pentahydrate to react with oxygen.

A preferred lower limit of the thickness of the electron-transporting layer 4 is 1 nm, and a preferred upper limit thereof is 2,000 nm. When such thickness is 1 nm or more, a hole can be sufficiently blocked, and when the thickness is 2,000 nm or less, the electron-transporting layer 4 is less liable to serve as a resistance at the time of the electron transportation, and hence the photoelectric conversion efficiency is improved. A more preferred lower limit of the thickness is 3 nm, a more preferred upper limit thereof is 1,000 nm, a still more preferred lower limit thereof is 5 nm, and a still more preferred upper limit thereof is 500 nm.

### <Application Examples>

Application examples of the present invention are directed to a photoelectric conversion apparatus, a moving body, and a building material.

### [Photoelectric Conversion Apparatus]

A photoelectric conversion apparatus of the present invention includes the photoelectric conversion element of the present invention. The photoelectric conversion apparatus may be formed by using the plurality of photoelectric conversion elements of the present invention. When the plurality of photoelectric conversion elements are connected, such photoelectric conversion apparatus may also be referred to as "photoelectric conversion cell" or "photoelectric conversion module." Photoelectric conversion elements having different absorption wavelengths may be laminated as the photoelectric conversion elements to increase an output voltage. In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor. In order to impart a function of, for example, maintaining or increasing the amount of incident light, a surface layer to which water or dirt is hard to adhere, or a function of collecting or guiding light may be added.

### [Moving Body]

A moving body of the present invention includes the photoelectric conversion element of the present invention. Fig. 2 is a perspective view for schematically illustrating a moving body including the photoelectric conversion element according to one embodiment of the present invention. A moving body 30 includes a photoelectric conversion element 31 of the present invention and a body 32 including the photoelectric conversion element 31. The photoelectric conversion element 31 is arranged on the position of the body 32 at which ambient light can be received. When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a motorcycle, a railway vehicle, a ship, or a flying body including an artificial satellite, an airplane, and a drone. The configuration of the body 32 of the moving body 30 is not particularly limited, but is preferably formed of a material having high strength.

### [Building Material]

A building material of the present invention includes the photoelectric conversion element of the present invention. Fig. 3 is a perspective view for schematically illustrating a building material including the photoelectric conversion element according to one embodiment of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. When the building material 40 is used for a roof or the like, the temperature of the photoelectric conversion element 41 may be increased by sunlight, and hence the photoelectric conversion efficiency may be reduced. The reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exterior 44a and the exterior 44b may be formed of the same member, or may be formed of different members. A paint or a transparent substrate may be used as each of the exteriors. An exterior having small light absorption and a high heat-shielding property is preferred.

### [Others]

In addition to the application examples described above, the following application examples are given: portable devices, such as a calculator, a sensor, and a small solar panel; wearable devices, such as a glasses-type terminal, a watch-type terminal, and a portable medical device; sheet structures supported by a plurality of frames, such as a tent, a plastic house, and a loading platform of a truck; and structures to be used by being fixed, such as a road surface panel, a floating panel, a building material utilizing the flexibility of a substrate, a wall-type building material, a glass-type building material, and a mega solar panel.

### [Method of producing Photoelectric Conversion Element]

A method of producing the photoelectric conversion element of the present invention includes the steps of: forming a first electrode; forming a second electrode; forming a photoelectric conversion layer arranged between the first electrode and the second electrode, the photoelectric conversion layer containing a crystal having a perovskite structure; and forming a charge-transporting layer between the photoelectric conversion layer and the first electrode.

The respective steps of the production method are described below.

### (Step of forming First Electrode and Step of forming Second Electrode)

The method of producing the photoelectric conversion element of the present invention includes the steps of: forming the first electrode; and forming the second electrode. In the step of forming the first electrode and the step of forming the second electrode, an appropriate method may be selected in accordance with a material for the first electrode and a material for the second electrode, respectively. Examples of such method include, but are not limited to, a sputtering method, a vacuum vapor deposition method, a vapor phase growth method (CVD method), and a spray pyrolysis deposition method (SPD method). The materials of the first electrode and the second electrode are as described above. When one, or each of both, of the first electrode and the second electrode is a transparent electrode, the thickness of the transparent electrode is preferably 0.03 to 3 µm.

When a solar cell is produced, cutting processing may be performed for circuit formation between steps. Examples of the cutting processing include mechanical patterning and laser patterning.

### (Modularization Step)

An element formed up to the electrode may be sealed. A sealing method is, for example, sealing with a resin or sealing with a film. Examples of a material used for the sealing include a silazane, a silicone rubber, a resin having a siloxane skeleton, and glass.

In addition, hairline treatment may be performed on the surface of the sealed element from the viewpoint of the suppression of adhesion between elements occurring during winding in a roll-to-roll system.

### (Step of forming Photoelectric Conversion Layer)

The step of forming the photoelectric conversion layer may include a step of applying a liquid containing the material for the photoelectric conversion layer described above. Examples of an application method include a spin coating method, a blade coating method, a slit die coating method, a screen printing method, a bar coater method, a casting method, a printing transfer method, a dip-up method, an ink jet method, a spray method, and a vacuum vapor deposition method. The method is appropriately selected therefrom in accordance with the properties of a photoelectric conversion layer to be produced, such as thickness control and orientation control.

Annealing treatment may be performed under reduced pressure or under an inert atmosphere (under a nitrogen or argon atmosphere) in order to remove a solvent or a dispersion medium from the applied liquid containing the material for the photoelectric conversion layer. The temperature of the annealing treatment is preferably 40 to 300°C, more preferably 50 to 150°C. The annealing treatment is preferably performed because materials for forming the respective layers may permeate each other at an interface between laminated layers to increase a contact area, and hence a short-circuit current can be increased.

### (Step of forming Charge-transporting Layer)

The step of forming the charge-transporting layer is preferably performed by a method of applying a resin solution in which the insulating resin is dissolved.

In addition, examples of the step of forming the charge-transporting layer include the following: a method including arranging the charge-transporting substance on the surface of the photoelectric conversion layer, and then applying the resin solution in which the insulating resin is dissolved; a method including applying the resin solution in which the insulating resin is dissolved on the surface of the photoelectric conversion layer, and then arranging the charge-transporting substance thereon; and a method including applying a solution, which is obtained by dispersing the charge-transporting substance in the resin solution in which the insulating resin is dissolved, onto the surface of the photoelectric conversion layer.

### [Examples]

The present invention is described in more detail below by way of Examples and Comparative Examples. The present invention is by no means limited to the following Examples without departing from the gist thereof. In the description of the following Examples, the term "part(s)" is by mass unless otherwise specified.

### <Production of Particle 1>

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4.5 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 71 mass%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times. Finally, the filter residue was freeze-dried to provide a hydroxygallium phthalocyanine particle (hydrous hydroxygallium phthalocyanine particle) having a solid content of 23 mass% in a yield of 71%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine (OHGaPc) particle (crystal) having a water content of 1.0 mass% or less was obtained.

### Step (3)

5 Parts of the hydroxygallium phthalocyanine particle was mixed with 5 parts of an N-methylformamide solvent, and the mixture was subjected to dispersion treatment for 6 hours with a sand mill (TSG-1/4G-4U, manufactured by Igarashi Machine Production Co., Ltd. (currently AIMEX Co., Ltd.), disc diameter: 70 mm, number of discs: 5) containing 5 parts of glass beads, followed by filtration and drying to provide a particle 1 (specific gravity: 1.6).

### <Production of Resin Solution 1>

1.0 Gram of polyvinyl butyral (product name: BM-2, manufactured by Sekisui Chemical Co., Ltd., specific gravity: 1.6) was dissolved in 19 g of 2-propanol by stirring for 24 hours to provide a resin solution 1.

### (Example 1)

### [Formation of Electron-transporting Layer]

A glass substrate with ITO was washed, and a tin(II) oxide colloidal solution (15% water dispersion, manufactured by Alfa Aesar) diluted fivefold was applied thereonto by spin coating, followed by heating at 150°C for 30 minutes to form an electron-transporting layer as a thin film having a thickness of 16 nm.

### [Formation of Photoelectric Conversion Layer]

0.487 Gram of methylammonium bromide, 1.034 g of formamidinium iodide, 2.903 g of lead iodide, and 0.139 g of methylammonium bromide were dissolved in 4.25 g of N,N-dimethylformamide and 1.216 g of dimethyl sulfoxide, and were stirred for 1 hour (solution 1). Further, 0.100 g of cesium iodide was dissolved in 0.285 g of dimethyl sulfoxide, and the solution was stirred for 1 hour (solution 2). After that, the cesium iodide solution (solution 2) was added to the solution 1 to prepare a photoelectric conversion layer coating liquid. The coating liquid was applied onto the electron-transporting layer by spin coating to form a photoelectric conversion layer formed of Cs_{0.05}(FA_{0.83}MA_{0.17})_{0.95}Pb(I_{0.83}Br_{0.17})₃ and having a thickness of 600 nm.

### [Formation of Charge-transporting Layer]

0.1 Gram of the particle 1 and 0.01 g of a calixarene compound (Japanese Patent Laid-Open No. 2003-207913) were mixed with 10.6 g of 2-propanol, and 11 g of beads (zirconia beads, Torayceram (trademark) zirconia beads, 0.3 mm) were loaded into the mixture, followed by paint shaker dispersion (manufactured by Toyo Seiki Co., Ltd.) for 3 hours. After that, 0.2 g of the resin solution 1 was added thereto, and paint shaker dispersion was performed again for 4 hours to prepare a charge-transporting layer solution. The charge-transporting layer solution was applied onto the photoelectric conversion layer by spin coating to form a charge-transporting layer having a thickness of 160 nm.

### [Introduction of Second Charge-transporting Layer]

0.15 Gram of Spiro-OMeTAD serving as a material for a second charge-transporting layer was dissolved in 2.2 g of chlorobenzene. 36 Microliters of an acetonitrile solution obtained by dissolving 0.2 g of lithium bis(trifluoromethanesulfonyl)imide in 0.3 g of acetonitrile and 60 µL of 4-tert-butylpyridine (TBP) were added to the chlorobenzene solution, and the contents were mixed. Further, 58 µL of an acetonitrile solution obtained by dissolving 0.11 g of [tris(2-(1H-pyrazol-1-yl)-4-tert-butylpyridine)cobalt(III) tris(bis(trifluoromethylsulfonyl)imide)] in 0.3 g of acetonitrile was mixed thereinto to prepare a material solution for a second charge-transporting layer. The material solution was applied onto the above-mentioned charge-transporting layer by a spin coating method to form a second charge-transporting layer having a thickness of 100 nm.

### [Formation of First Electrode]

A gold electrode having a thickness of 80 nm and an area of 0.09 cm² was formed on the second charge-transporting layer by a vacuum vapor deposition method. Thus, a photoelectric conversion element was obtained.

### [Analysis of Amount of Compound]

The electrode surface of the photoelectric conversion element was peeled off to expose the surface of the charge-transporting layer. The surface of the charge-transporting layer was wiped with a cotton swab or the like with a solvent, dissolved in deuterated sulfuric acid, and subjected to ¹H-NMR measurement (apparatus: AVANCE 3-500 manufactured by BRUKER). In addition, the mass and structure analysis of the peeled-off charge-transporting layer components was performed by elemental analysis, such as GPC and MALDI-TOF-MS, IR, gas chromatography, XPS, and EDX, to recognize the presence of a compound.

In addition, the thickness of the charge-transporting layer was observed with a cross-sectional SEM (apparatus: SmartSEM manufactured by Carl Zeiss Co., Ltd.) after the cutting of the photoelectric conversion element and the fixing of the sample to a tilted sample stage.

### [Analysis of Particle Diameter of Charge-transporting Substance]

The particle diameter of a charge-transporting substance is a number-average particle diameter in a particle size distribution. In the present invention, the particle diameter of the charge-transporting substance was derived by an image imaging method using a TEM.

Specifically, first, N particles (N represents 1,000 or more) were extracted by using image processing software Photoshop (manufactured by Adobe Inc.) through use of a TEM image of the resultant cross section of the charge-transporting layer. Next, the area S of each particle was determined, the diameter of a circle with the same area as the area (=2×(S/π)^{1/2}) was defined as the particle diameter, and an average value of the central 80% of the N particles was adopted.

### (Example 2)

A photoelectric conversion element is obtained in the same manner as in Example 1 except that the ratio of the volume of the charge-transporting substance to the volume of the insulating resin is set to 8.

### (Example 3)

A photoelectric conversion element is obtained in the same manner as in Example 1 except that the ratio of the volume of the charge-transporting substance to the volume of the insulating resin is set to 15.

### (Example 4)

A photoelectric conversion element is obtained in the same manner as in Example 1 except that, in the formation of the charge-transporting layer, the dispersion liquid obtained by the paint shaker dispersion is further centrifuged (15,000 rpm, 6 minutes) so that the particle diameter in the dispersion may be reduced, followed by the adjustment of the ratio of the volume of the charge-transporting substance to the volume of the insulating resin to 10.

### (Example 5)

A photoelectric conversion element is obtained in the same manner as in Example 1 except that the polyvinyl butyral (product name: BM-2, manufactured by Sekisui Chemical Co., Ltd.) is changed to polyvinyl butyral (product name: BX-1, manufactured by Sekisui Chemical Co., Ltd.).

### (Example 6)

A photoelectric conversion element is obtained in the same manner as in Example 1 except that the calixarene compound is changed to 2-naphthol.

### (Example 7)

A photoelectric conversion element is obtained in the same manner as in Example 1 except that the particle 1 is changed to a copper phthalocyanine particle.

### (Example 8)

A photoelectric conversion element is obtained in the same manner as in Example 1 except that the second charge-transporting layer is not arranged.

### (Example 9)

A photoelectric conversion element is obtained in the same manner as in Example 1 except that the calixarene compound is not used.

### (Example 10)

A photoelectric conversion element is obtained in the same manner as in Example 1 except that the polyvinyl butyral is changed to polymethyl methacrylate (PMMA, manufactured by Sigma-Aldrich Co. LLC, glass transition temperature: 70°C).

### (Example 11)

A photoelectric conversion element is obtained in the same manner as in Example 1 except that the polyvinyl butyral is changed to polymethyl methacrylate (PMMA, manufactured by Sigma-Aldrich Co. LLC, glass transition temperature: 100°C).

### (Example 12)

A photoelectric conversion element is obtained in the same manner as in Example 1 except that the particle 1 is changed to a particle containing a compound represented by the following formula (Pc-3).

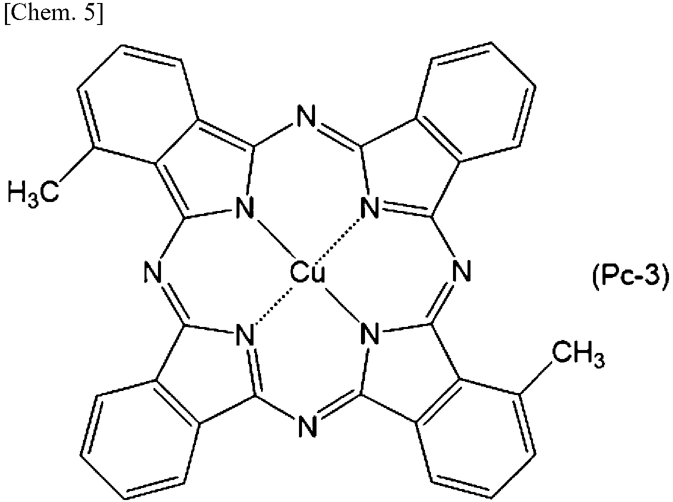

### (Example 13)

A photoelectric conversion element is obtained in the same manner as in Example 1 except that the particle 1 is changed to a quinacridone particle.

### (Example 14)

A photoelectric conversion element is obtained in the same manner as in Example 1 except that the ratio of the volume of the charge-transporting substance to the volume of the insulating resin is set to 20.

### (Example 15)

A photoelectric conversion element was obtained in the same manner as in Example 1 except that the composition of the photoelectric conversion layer is changed to MAPbI₃.

### (Comparative Example 1)

A photoelectric conversion element was obtained in the same manner as in Example 1 except that the ratio of the volume of the charge-transporting substance to the volume of the insulating resin is set to 2.

### (Comparative Example 2)

A photoelectric conversion element was obtained in the same manner as in Example 1 except that the insulating resin is not used.

### (Comparative Example 3)

A photoelectric conversion element was obtained in the same manner as in Example 11 except that: the particle 1 is changed to SPIRO-OMeTAD; and the second charge-transporting layer is not arranged.

### (Comparative Example 4)

A photoelectric conversion element is obtained in the same manner as in Example 1 except that: the particle 1 is changed to a nickel(II) phthalocyanine-tetrasulfonic acid tetrasodium salt particle; and the insulating resin is changed to PEDOT:PSS.

### (Comparative Example 5)

A photoelectric conversion element is obtained in the same manner as in Example 1 except that: the particle 1 is changed to a particle containing a compound represented by the following formula (Pc-4); and P3HT, which is a conductive resin, is used instead of the insulating resin.

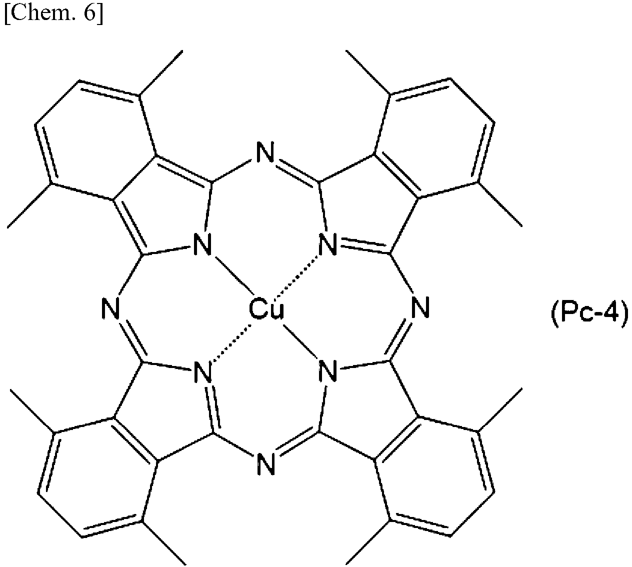

### (Comparative Example 6)

A photoelectric conversion element is obtained in the same manner as in Example 1 except that: the particle 1 is changed to a carbon nanotube (multi-wall-type carbon nanotube); and the insulating resin is changed to a polycarbonate resin (product name: PCZ-200, manufactured by Mitsubishi Gas Chemical Company, Inc.).

### (Comparative Example 7)

A photoelectric conversion element is obtained in the same manner as in Example 1 except that the particle 1 is changed to the following formula (H-1); and the insulating resin is changed to a polycarbonate resin (PCZ-200).

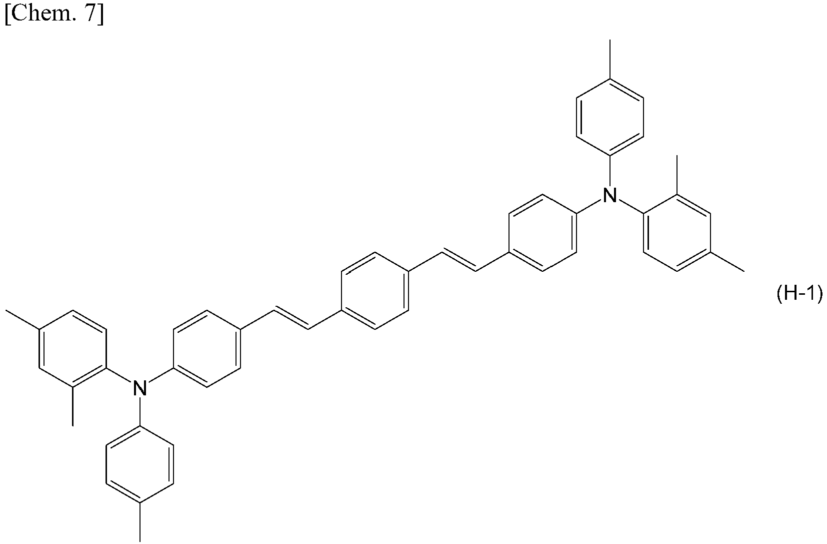

### [Evaluation]

The surface of the charge-transporting layer produced in Example 1 was observed with an optical microscope, and values of D and P were determined. The results are shown in Table 2. A "color" mode was used as the CCD mode of the optical microscope. White light used for the light source of the optical microscope is a xenon lamp (LC8 manufactured by Hamamatsu Photonics K.K.). A color image observed with the optical microscope was imported into a personal computer, 8-bit grayscale processing in an OpenCV library (Ver. 4.8.1) was performed on Python (trademark), and Otsu's binarization processing was performed based on pixel values as described above to determine values of D and P.

Next, a power supply (236 model, manufactured by Keithley Instruments) was connected between the electrodes of the photoelectric conversion element produced in Example 1, and its photoelectric conversion efficiency was measured by: irradiating the element with constant light through use of a solar simulator (manufactured by Yamashita Denso Corporation) having an intensity of 110 mW/cm²; and measuring the generated current and voltage. After that, light of 10,000 Lx was continuously applied to the element with a white LED, and its photoelectric conversion efficiency after 60 days was measured. Then, the maintenance rate of the photoelectric conversion efficiency after 60 days with respect to the initial photoelectric conversion efficiency thus obtained was evaluated. The results are shown in Table 2.

The photoelectric conversion elements of Examples 2 to 15 and Comparative Examples 1 to 7 are each evaluated for its maintenance rate of the photoelectric conversion efficiency in the same manner as in Example 1. The results are shown in Table 2.

In each of Comparative Examples 3 to 7, no phase separation was observed in the charge-transporting layer, and the particle diameter could not be measured.

### [Table 2]

**Table 2**

| | Charge-transporting material | | Insulating resin | | Volume ratio of charge-transporting material with respect to insulating resin |
|---|---|---|---|---|---|
| | Compound | Particle diameter [nm] | Compound | Glass transition temperature [°C] | |
| Example 1 | Hydroxygallium phthalocyanine | 2.2×10² | BM-2 | 71°C | 10 |
| Example 2 | Hydroxygallium phthalocyanine | 1.9×10² | BM-2 | 71°C | 8 |
| Example 3 | Hydroxygallium phthalocyanine | 2.6×10² | BM-2 | 71°C | 15 |
| Example 4 | Hydroxygallium phthalocyanine | 15 | BM-2 | 71°C | 10 |
| Example 5 | Hydroxygallium phthalocyanine | 2.1×10² | BX-1 | 95°C | 10 |
| Example 6 | Hydroxygallium phthalocyanine | 2.9×10² | BM-2 | 71°C | 10 |
| Example 7 | Copper phthalocyanine | 3.1×10² | BM-2 | 71°C | 10 |
| Example 8 | Hydroxygallium phthalocyanine | 2.2×10² | BM-2 | 71°C | 10 |
| Example 9 | Hydroxygallium phthalocyanine | 3.5×10² | BM-2 | 71°C | 10 |
| Example 10 | Hydroxygallium phthalocyanine | 2.7×10² | PMMA | 70°C | 10 |
| Example 11 | Hydroxygallium phthalocyanine | 2.9×10² | PMMA | 100°C | 10 |
| Example 12 | (Pc-3) | 36 | BM-2 | 71°C | 10 |
| Example 13 | Quinacridone | 1.3×10² | BM-2 | 71°C | 10 |
| Example 14 | Hydroxygallium phthalocyanine | 3.8×10² | BM-2 | 71°C | 20 |
| Example 15 | Hydroxygallium phthalocyanine | 2.2×10² | BM-2 | 71°C | 10 |
| Comparative Example 1 | Hydroxygallium phthalocyanine | 1.8×10² | BM-2 | 71°C | 2 |
| Comparative Example 2 | Hydroxygallium phthalocyanine | 3.7×10² | Absent | - | - |
| Comparative Example 3 | SPIRO-OMeTAD | - | PMMA | 100°C | 10 |
| Comparative Example 4 | Nickel(II) phthalocyanine-tetrasulfonic acid tetrasodium salt | - | PEDOT:PSS | - | 10 |
| Comparative Example 5 | (Pc-4) | - | P3HT | 174°C | 10 |
| Comparative Example 6 | Carbon nanotube | - | PCZ-200 | 174°C | 10 |
| Comparative Example 7 | (H-1) | - | PCZ-200 | 174°C | 10 |

**Table 2 (continued)**

| | Aromatic ring compound having hydroxy group | Second charge-transporting layer | D [µm] | P | Maintenance rate of photoelectric conversion efficiency [%] |
|---|---|---|---|---|---|
| Example 1 | Calixarene compound | Present | 6.6 | 7.6 | 95.2 |
| Example 2 | Calixarene compound | Present | 6.7 | 6.5 | 96.8 |
| Example 3 | Calixarene compound | Present | 5.5 | 7.6 | 94.8 |
| Example 4 | Calixarene compound | Present | 4.2 | 5.1 | 95.3 |
| Example 5 | Calixarene compound | Present | 6.8 | 7.3 | 93.8 |
| Example 6 | 2-Naphthol | Present | 7 | 7 | 92.7 |
| Example 7 | Calixarene compound | Present | 7.3 | 6.2 | 90.9 |
| Example 8 | Calixarene compound | Absent | 6.1 | 5.5 | 86.6 |
| Example 9 | Absent | Present | 7.4 | 6 | 87.2 |
| Example 10 | Calixarene compound | Present | 7 | 6.9 | 89.1 |
| Example 11 | Calixarene compound | Present | 7.1 | 6.7 | 82.5 |
| Example 12 | Calixarene compound | Present | 4.7 | 5.4 | 87.9 |
| Example 13 | Calixarene compound | Present | 5.3 | 5.7 | 83.3 |
| Example 14 | Calixarene compound | Present | 5.5 | -8.8 | 89.3 |
| Example 15 | Calixarene compound | Present | 6.6 | -7.6 | 94.6 |
| Comparative Example 1 | Calixarene compound | Present | 11.5 | 8 | 76.8 |
| Comparative Example 2 | Calixarene compound | Present | 6.5 | 8.5 | 72.2 |
| Comparative Example 3 | Calixarene compound | Present | 2.9 | 4.7 | 70.6 |
| Comparative Example 4 | Calixarene compound | Present | 3.3 | 4.8 | 71.3 |
| Comparative Example 5 | Calixarene compound | Present | 3.4 | 4.7 | 73.9 |
| Comparative Example 6 | Calixarene compound | Present | 3.8 | 5.1 | 70.7 |
| Comparative Example 7 | Calixarene compound | Present | 2.5 | 4.5 | 71.2 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, Japanese Patent Application No. 2024-085999 filed on May 28, 2024, and Japanese Patent Application No. 2024-186505 filed on October 23, 2024, and the entire contents thereof are incorporated herein by reference.

### [Reference Signs List]

1 photoelectric conversion element
2 substrate
3 second electrode
4 electron-transporting layer
5 photoelectric conversion layer
6 charge-transporting layer
7 first electrode
30 moving body
31, 41 photoelectric conversion element
32 body
40 building material
42 protective member
43 heat dissipation member
44a, 44b exterior

## Claims

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element comprises a charge-transporting layer between the photoelectric conversion layer and the first electrode,
wherein the charge-transporting layer contains a charge-transporting substance and an insulating resin that are phase-separated from each other,
wherein a ratio of a volume of a region of the charge-transporting substance to a volume of a region of the insulating resin is 5 to 30, and
wherein, in a binarized image of a two-dimensional spatial distribution of a region A and a region B obtained by measuring the charge-transporting layer with an optical microscope, when a value obtained by Fourier analysis of the binarized image, the value being determined by the following method of calculating D, is represented by D [µm], the D [µm] satisfies the following formula (E1). $4 \leq D \leq 12$ <Method of calculating D>
With regard to the region A and the region B, an image of a two-dimensional spatial distribution having a size of L [µm]×L [µm], where L [µm] is 100 µm or more, is binarized by Otsu's binarization method as shown in the following formula (E3):
[Math. 1] $c \left[m, n\right] = \left\{\begin{matrix}1 & for region A \\ 0 & for region B\end{matrix}\right.$
where, for discrete data of N×N pixels obtained by dividing L into N with an even number N of 1,024 or more, "m" and "n" each represent an integer of -N/2 to N/2-1, and each represent a coordinate in the two-dimensional spatial distribution.
Next, c[m, n] obtained by the formula (E3) is subjected to discrete Fourier transform in accordance with the following formula (E4), where "k" and "l" each represent an integer of -N/2 to N/2.
[Math. 2] $C \left[k, l\right] = {\sum }_{m = - N / 2}^{N / 2 - 1} {\sum }_{n = - N / 2}^{N / 2 - 1} c \left[m, n\right] {e}^{- i \frac{2 π}{N} \left(km+ln\right)}$
Then, a domain size D [µm] is calculated in accordance with the following formula (E5) by using C[k, l] obtained by the formula (E4).
[Math. 3] $D = \frac{L \left[{\sum }_{{k , l = - \frac{N}{2}}_{\left(k, l\right) \neq \left(0, 0\right)}}^{\frac{N}{2} - 1} \frac{C \left[k, l\right] C \left[-k,-l\right]}{\sqrt{{k}^{2} + {l}^{2}}}\right]}{\left[{\sum }_{{k , l = - \frac{N}{2}}_{\left(k, l\right) \neq \left(0, 0\right)}}^{\frac{N}{2} - 1} \left(C\left[k, l\right]C\left[-k,-l\right]\right)\right]}$

2. The photoelectric conversion element according to claim 1, wherein, in the binarized image of the two-dimensional spatial distribution of the region A and the region B obtained by measuring the charge-transporting layer with the optical microscope, when a value obtained by Fourier analysis of the binarized image, the value being determined by the following method of calculating P, is represented by P, the P satisfies the following formula (E2). $8.0 \leq P \leq 9.7$ <Method of calculating P>
An order parameter P is calculated in accordance with the following formula (E6) by using C[k, l] obtained by the formula (E4).
[Math. 4] $P = - {\sum }_{{k , l = - N / 2}_{\left(k, l\right) \neq \left(0, 0\right)}}^{N / 2 - 1} \left\{\left[\begin{matrix}C \left[k, l\right] C \left[-k,-l\right] \\ / \left({\sum }_{{k , l = - N / 2}_{\left(k, l\right) \neq \left(0, 0\right)}}^{N / 2 - 1} \left(C\left[k, l\right]C\left[-k,-l\right]\right)\right)\end{matrix}\right]ln\left[\begin{matrix}C \left[k, l\right] C \left[-k,-l\right] \\ / \left({\sum }_{{k , l = - N / 2}_{\left(k, l\right) \neq \left(0, 0\right)}}^{N / 2 - 1} \left(C\left[k, l\right]C\left[-k,-l\right]\right)\right)\end{matrix}\right]\right\}$

3. The photoelectric conversion element according to claim 1 or 2, wherein the charge-transporting substance is a pigment having a particle diameter of 1.0×10¹ to 5.0×10² nm.

4. The photoelectric conversion element according to any one of claims 1 to 3, wherein the charge-transporting substance is a phthalocyanine compound.

5. The photoelectric conversion element according to claim 4, wherein the phthalocyanine compound has a structure represented by the following formula (Pc-2): 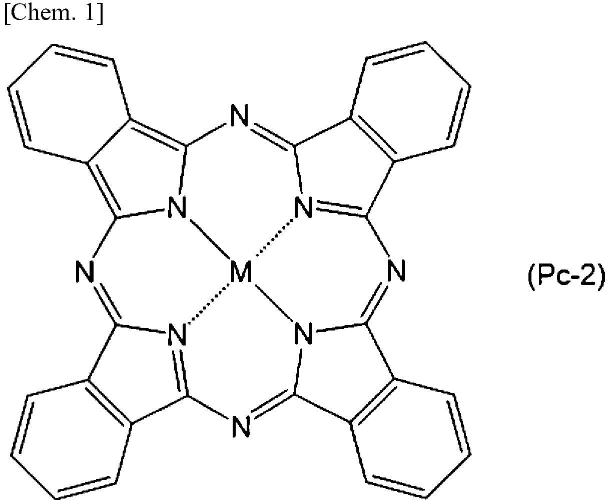 where M in the formula (Pc-2) represents H₂, a metal atom having a ligand, or a metal atom free of a ligand.

6. The photoelectric conversion element according to any one of claims 1 to 5, wherein the insulating resin has a glass transition temperature of 95°C or less.

7. The photoelectric conversion element according to any one of claims 1 to 6, wherein the insulating resin is a polyvinyl acetal resin or a polyvinyl butyral resin.

8. The photoelectric conversion element according to claim 3, wherein the charge-transporting layer contains an aromatic ring compound having a hydroxy group, the aromatic ring compound being different from the pigment and the insulating resin.

9. The photoelectric conversion element according to any one of claims 1 to 8, wherein the photoelectric conversion element further comprises a second charge-transporting layer between the first electrode and the charge-transporting layer.

10. A photoelectric conversion apparatus comprising the photoelectric conversion element of any one of claims 1 to 9.
